# EUROPÄISCHE PATENTSCHRIFT

(11) **EP 0 661 262 B1**
(45) Veröffentlichungstag und Bekanntmachung des Hinweises auf die Patenterteilung: **23.09.1998**
(21) Anmeldenummer: 95101833.2
(22) Anmeldetag: 20.10.1990
(51) Int. Cl.: C07C 249/12

(54) **Halogenierte Triazolinone**
Halogenated Triazolinones
Triazolinones halogénées

(30) Priorität: 03.11.1989 DE 3936622
(43) Veröffentlichungstag der Anmeldung: 05.07.1995
(62) Teilanmeldung aus: 90120152.5
(73) Patentinhaber: BAYER AG, 51368 Leverkusen (DE)
(72) Erfinder: Müller, Dr. Klaus-Helmut, D-40593 Düsseldorf (DE); Babczinski, Dr. Peter, D-42115 Wuppertal (DE); Santel, Dr. Hans-Joachim, D-51371 Leverkusen (DE); Schmidt, Dr. Robert R., D-51467 Bergisch Gladbach (DE)

(56) Entgegenhaltungen:
- DE-A- 3 920 414
- CHEMISCHE BERICHTE, Bd. 102,Nr. 3, 1969 WEINHEIM, D, Seiten 755-765, C.-F. KRÖGER, ET AL.: 'Über 1.2.4-Triazole, XVII. Die Nitrierung und Bromierung von 1.2.4-Triazolonen '

## Beschreibung

Die Erfindung betrifft neue halogenierte Triazolinone, welche als Zwischenprodukte zur Herstellung von neuartigen, herbizid wirksamen halogenierten Sulfonylaminocarbonyltriazolinonen verwendet werden können.

Es ist bereits bekannt, daß bestimmte 2-Aminocarbonyl-5-(amino- oder -mercapto)-2,4-dihydro-3H-1,2,6-triazol-3-one, die in 4-Stellung durch unterschiedliche Reste substituiert sein können, herbizid wirksam sind; ebenso sind Verfahren zu deren Herstellung und dafür erforderliche Zwischenprodukte, nämlich entsprechende, aber in 2-Stellung unsubstituierte 5-(Amino- oder -mercapto)-2,4-dihydro-3H-1,2,6-triazol-3-one, bekannt (vgl. EP-A-283 876).

Auch einige in 2-Stellung unsubstituierte, in 4-Stellung lediglich durch Methyl, Benzyl oder Phenyl substituierte 5-Halogen-2,4-dihydro-3H-1,2,4-triazol-3-one sind aus der chemischen Literatur vorbekannt. Im einzelnen handelt es sich hierbei um die fünf Verbindungen 4-Methyl-5-brom-, 4-Benzyl-5-brom-, 4-Phenyl-5-brom-, 6-Methyl-5-chlor- und 4-Benzyl-5-chlor-2,4-dihydro-3H-1,2,4-triazol-3-on, für die jedoch keine Verwendung beschrieben wird (vgl. Kröger et al., Chemische Berichte,Bd. 102, Nr. 3, 1969, Seiten 759 - 760).

Es wurden jetzt die neuen 4-substituierten 5-Halogen-2,4-dihydro-3H-1,2,4-triazol-3-one der Formel (II) aufgefunden, 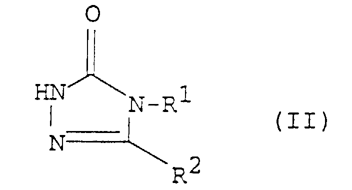 in welcher
- R¹: für C₃-C₆-Alkenyl, für gegebenenfalls durch C₁-C₄-Alkyl substituiertes C₃-C₆-Cycloalkyl, für C₁-C₆-Alkoxy, für C₁-C₄-Alkylamino oder für Di-(C₁-C₄-alkyl)-amino steht und
- R²: für Fluor, Chlor, Brom oder Jod steht.

Diese neuen Verbindungen der Formel (II) werden oben wie auch nachfolgend als "halogenierte Triazolinone" bezeichnet.

Die neuen halogenierten Triazolinone der Formel (II) können nach an sich bakannten Verfahren hergestellt werden (vgl. den oben bereits zitierten Artikel von Kröger et al., Chem. Ber. 102 (1969), S. 755-766).

Wie weiter gefunden wurde, können die halogenierten Triazolinone der Formel (II) nach mehreren Verfahren zu neuartigen, in 4-Stellung entsprechend substituierten 2-Sulfonylaminocarbonyl-5-halogen-2,4-dihydro-3H-1,2,4-triazol-3-onen der Formel (I) 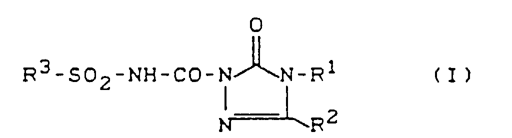 in welcher
- R¹ und R²: die oben bei Formel (II) angegebenen Bedeutungen haben und
- R³: für einen gegebenenfalls substituierten Rest aus der Reihe Alkyl, Aralkyl, Aryl und Heteroaryl steht, wobei jeweils die exakten Definitionen dieser Reste und ihrer möglichen Substituenten gemäß dem auf die zugehörige Stammanmeldung erteilten EP-Patent 0 425 948 gelten,
und deren Salzen umgesetzt werden, die - wie außerdem gefunden wurde - wertvolle herbizide Eigenschaften besitzen.

Die Endprodukte der Formel (I), die nachfolgend aufgeführten Verfahren zu ihrer Herstellung und ihre Verwendung als Herbizide sind Gegenstand der zugehörigen Stammanmeldung (jetzt EP-Patent Nr. 0 425 948).

Die Endprodukte der Formel (I) können hergestellt werden, indem man
a) halogenierte Triazolinone der allgemeinen Formel (II) 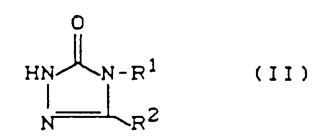 in welcher
   - R¹ und R²: die oben angegebenen Bedeutungen haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Verdünnungsmittels umsetzt, oder wenn man
b) halogenierte Triazolinon-Derivate der allgemeinen Formel (IV) 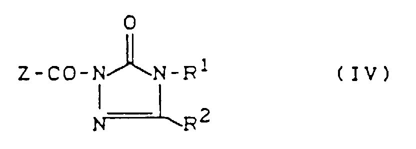 in welcher
   - R¹ und R²: die oben angegebenen Bedeutungen haben und
   - Z: für Chlor, C₁-C₄-Alkoxy, Benzyloxy oder Phenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt, oder wenn man
c) halogenierte Triazolinone der allgemeinen Formel (II) 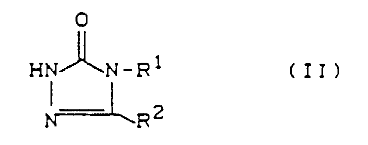 in welcher
   - R¹ und R²: die oben angegebenen Bedeutungen haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Chlor, C₁-C₄-Alkoxy, Benzyloxy oder Phenoxy steht,
   gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.
   Nähere Angaben zu den einzelnen Herstellungsverfahren (a), (b) und (c) - das heißt zu den Verfahrensbedingungen und den übrigen hierbei einzusetzenden Zwischenprodukten sowie gegebenenfalls deren Herstellung - sind dem oben bereits genannten EP-Patent 0 425 948 zu entnehmen.
   Bevorzugte Verbindungen der Formel (II) sind solche, bei denen
   - R¹: für Allyl, für C₃-C₆-Cycloalkyl, für C₁-C₄-Alkoxy, für C₁-C₃-Alkylamino oder für Di-(C₁-C₃-alkyl)-amino steht und
   - R²: für Chlor oder Brom steht.

Besonders bevorzugte Verbindungen der Formel (II) sind diejenigen, bei denen

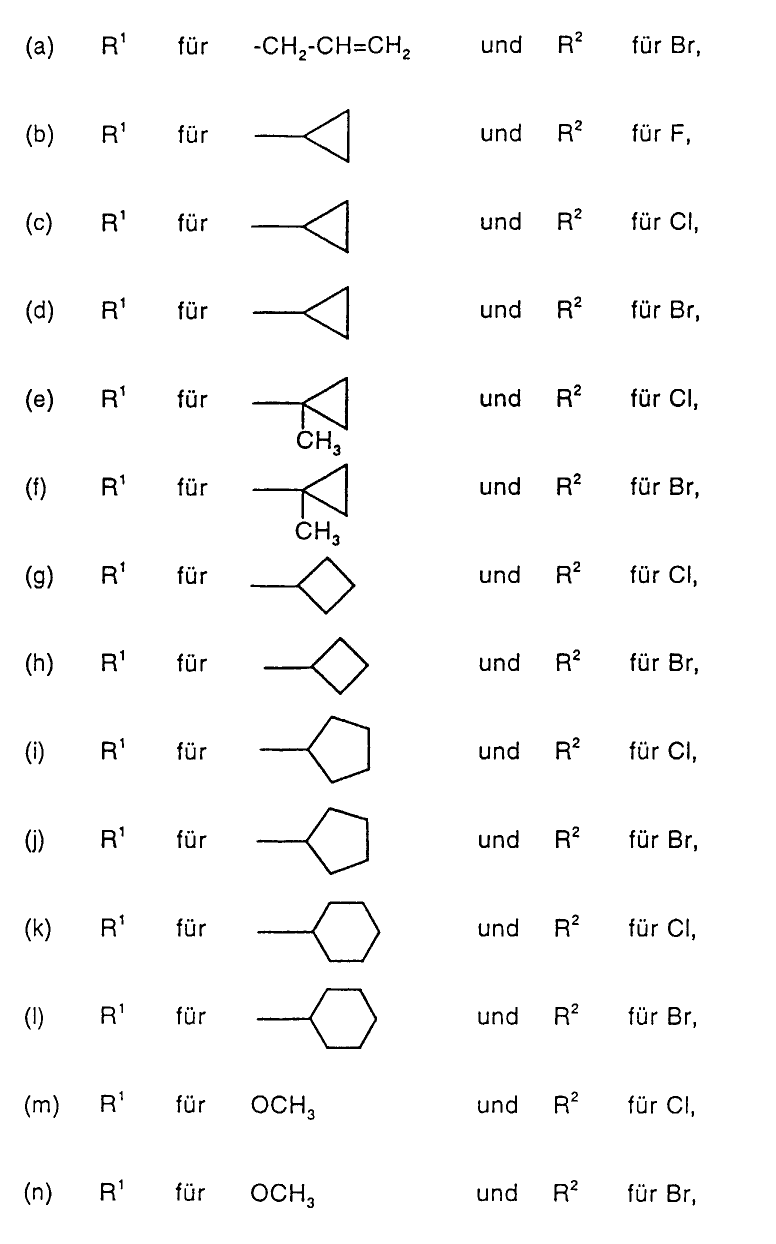

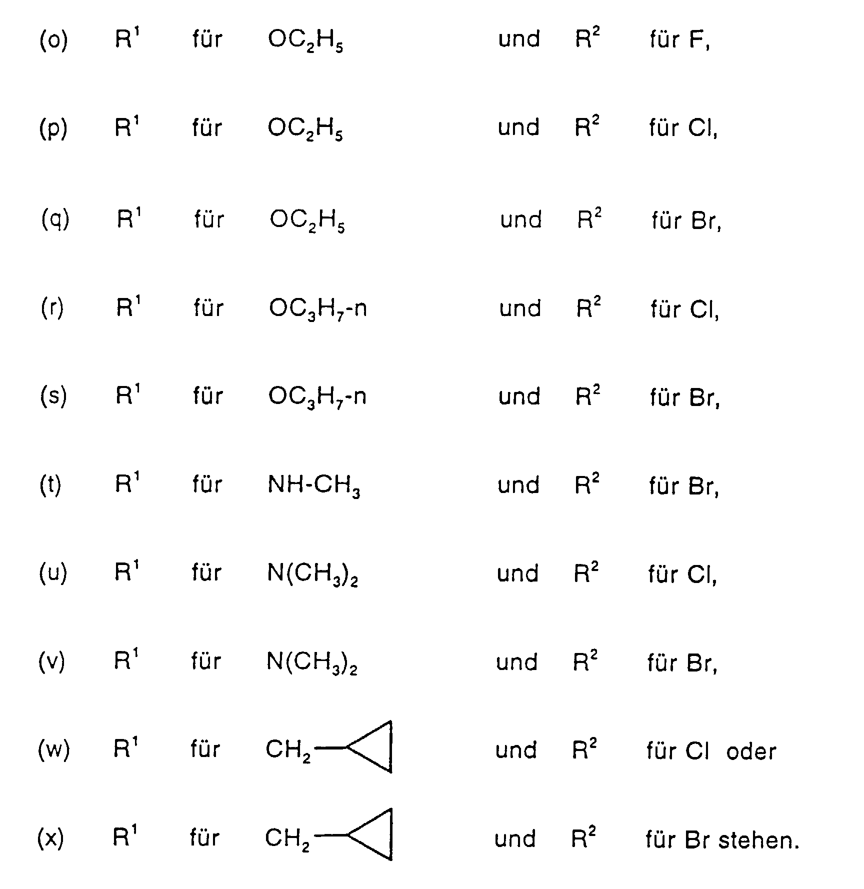

Die Herstellung der erfindungsgemäßen halogenierten Triazolinone der obigen Formel (II) wird in den nachfolgenden Beispielen näher erläutert.

### Herstellungsbeispiele

### Beispiel (II-1)

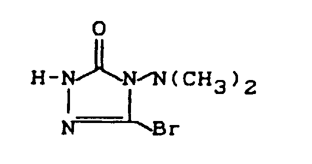

### Stufe 1:

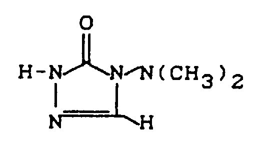

856 g (4,0 Mol) Diphenylcarbonat werden in 588 g Ethylenchlorid gelöst. Unter Wasserkühlung werden 245 g (4,0 Mol) Dimethylhydrazin (98%ig) zugetropft, dann wird langsam erwärmt und nach 4 Stunden bei 60°C nachgerührt.

Nach Abkühlen auf 20°C werden 200 g (4,0 Mol) Hydrazinhydrat zugetropft und 12 Stunden nachgerührt. Es wird auf 70-80°C erwärmt und nach ca. 1 Stunde weiter gerührt. Die erkaltete Lösung wird im Vakuum destilliert, wobei Ethylenchlorid und Wasser entfernt werden (Sumpftemperatur zuletzt 100°C). Zu 424 g (4,0 Mol) Orthoameisensäure-trimethylester wird im Verlauf von 90 Minuten bei Rückflußtemperatur (ca. 102°C) obige phenolische Dimethylcarbodihydrazid-Lösung getropft. Nach destillativem Entfernen des gebildeten Methanols wird im Vakuum Phenol abdestilliert, anschließend werden bei einer Kopftemperatur von 85-105°C 282 g Produktgemisch erhalten. Dieses Gemisch wird mit 600 ml Aceton verkocht und nach Filtration in der Siedehitze wird das Filtrat abgekühlt. Das dabei kristallin angefallene Produkt wird durch Absaugen isoliert.

Man erhält 71 g (14% der Theorie) 4-Dimethylamino-2,4-dihydro-3H-1,2,4-triazol-3-on vom Schmelzpunkt 127°C.

### Stufe 2:

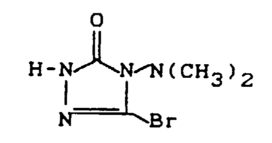

Eine Mischung aus 15,0 g (0,117 Mol) 4-Dimethylamino-2,4-dihydro-3H-1,2,4-triazol-3-on, 4,7 g (0,117 Mol) Natriumhydroxid und 150 ml Waser wird unter Eiskühlung und unter Rühren im Verlauf von 2 Stunden mit 18,8 g (0,117 Mol) Brom versetzt. Das kristallin angefallene Produkt wird anschließend durch Absaugen isoliert.

Man erhält 19,6 g (81% der Theorie) 5-Brom-4-dimethylamino-2,4-dihydro-3H-1,2,4-triazol-3-on vom Schmelzpunkt 163°C.

### Beispiel (II-2)

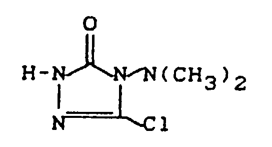

Eine Mischung aus 6,0 g (0,029 Mol) 5-Brom-4-dimethylamino-2,4-dihydro-3H-1,2,4-triazol-3-on und 200 ml konzentrierter Salzsäure wird 3 Stunden unter Rückfluß erhitzt. Dann wird eingeengt, der Rückstand in wenig Wasser aufgenommen, mit Natriumhydrogencarbonat neutralisiert und das kristallin angefallene Produkt durch Absaugen isoliert. Das Filtrat wird mit Essigsäureethylester extrahiert, das vorher isolierte kristalline Produkt zur organischen Phase gegeben, letztere mit Natriumsulfat getrocknet und filtriert. Das Filtrat wird eingeengt, der Rückstand mit Diethylether verrieben und das hierbei kristallin angefallene Produkt durch Absaugen isoliert.

Man erhalt 3,1 g (66 % der Theorie) 5-Chlor-4-dimethylamino-2,4-dihydro-3H-1,2,4-triazol-3-on vom Schmelzpunkt 158°C.

Analog zu den Beispielen (II-1) unds (II-2) können beispielsweise auch die in der nachstehenden Tabelle 4 aufgeführten Verbindungen der Formel (II) hergestellt werden.

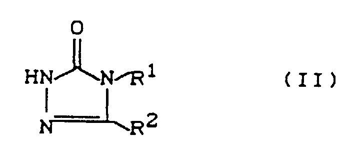

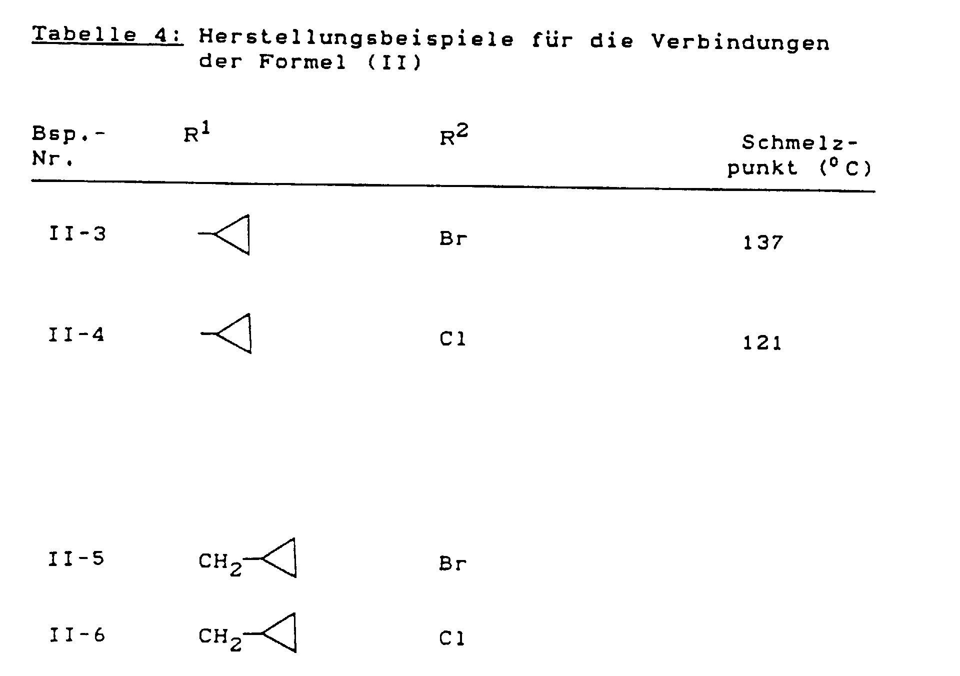

## Patentansprüche

1. Halogenierte Triazolinone der Formel (II), 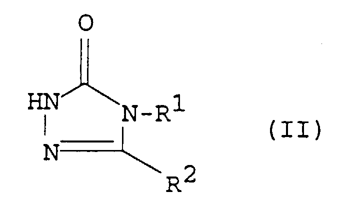 in welcher
R¹ für C₃-C₆-Alkenyl, für gegebenenfalls durch C₁-C₄-Alkyl substituiertes C₃-C₆-Cycloalkyl, für C₁-C₆-Alkoxy, für C₁-C₄-Alkylamino oder für Di-(C₁-C₄-alkyl)-amino steht und
R² für Fluor, Chlor, Brom oder Jod steht.

2. Halogenierte Triazolinone der Formel (II) gemäß Anspruch 1, dadurch gekennzeichnet, daß darin
R¹ für Allyl, für C₃-C₆-Cycloalkyl, für C₁-C₄-Alkoxy, für C₁-C₃-Alkylamino oder für Di-(C₁-C₃-alkyl)-amino steht und
R² für Chlor oder Brom steht.

3. Halogenierte Triazolinone der Formel (II) 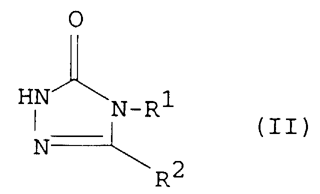 in welcher

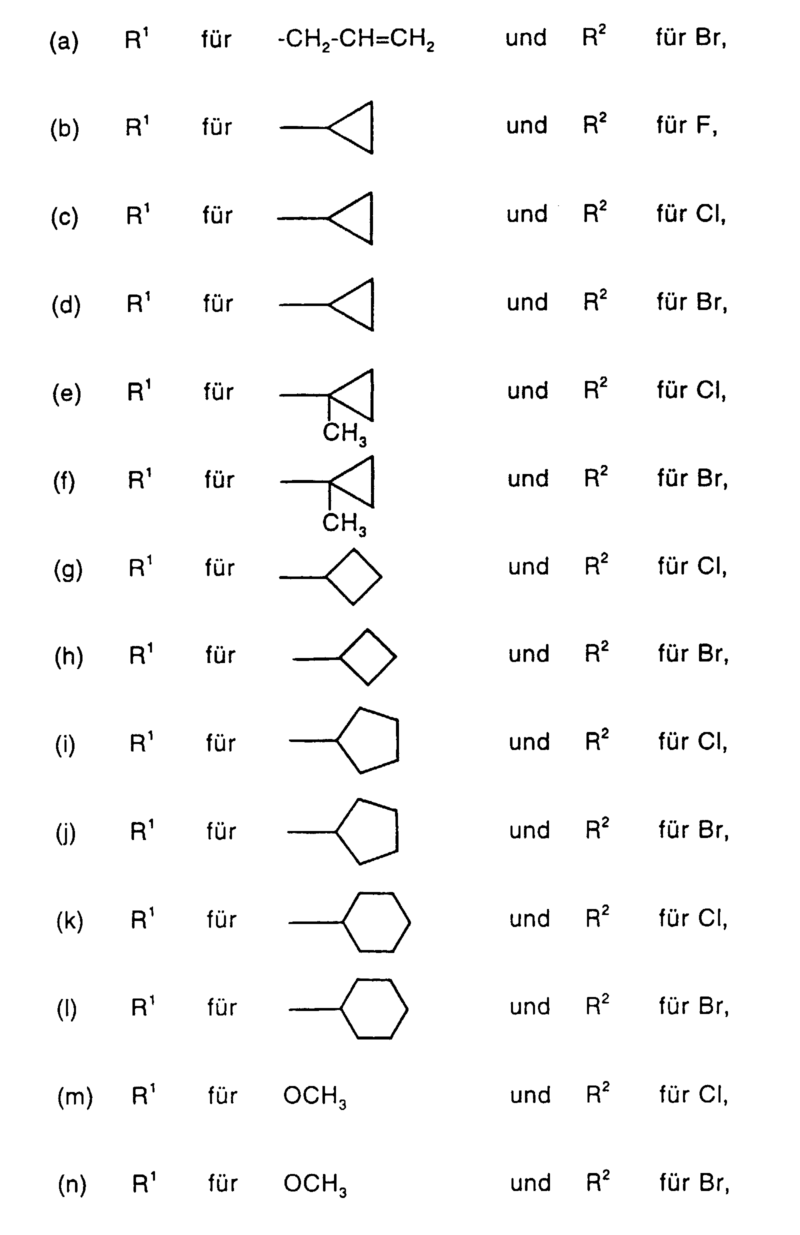

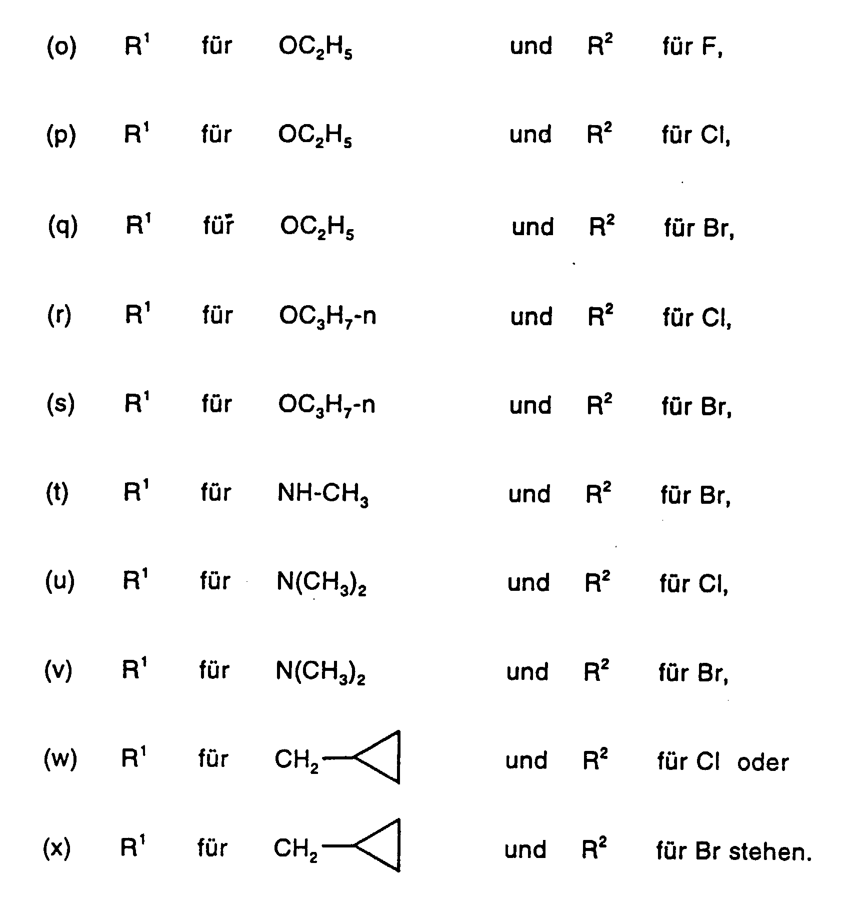

## Claims

1. Halogenated triazolinones of the formula (II) 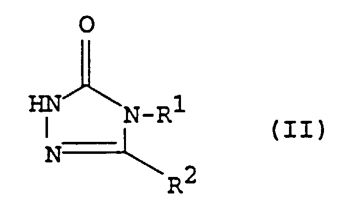 in which
R¹ represents C₃-C₆-alkenyl, or represents C₃-C₆-cycloalkyl which is optionally substituted by C₁-C₄-alkyl, or represents C₁-C₆-alkoxy, or represents C₁-C₄-alkylamino, or represents di- (C₁-C₄-alkyl) -amino and
R² represents fluorine, chlorine, bromine or iodine.

2. Halogenated triazolinones of the formula (II) according to Claim 1, characterized in that, in these halogenated triazolinones,
R¹ represents allyl, C₃-C₆-cycloalkyl, C₁-C₄-alkoxy, C₁-C₃-alkylamino or di-(C₁-C₃-alkyl)-amino and
R² represents chlorine or bromine.

3. Halogenated triazolinones of the formula (II) 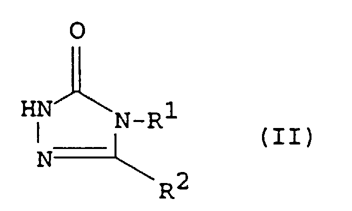 in which

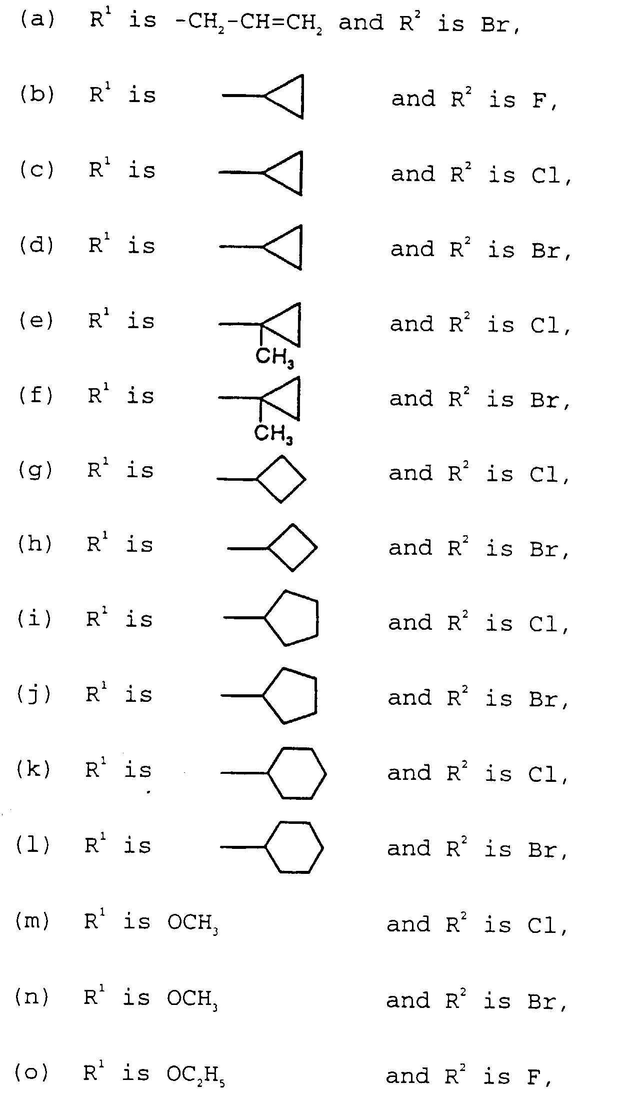

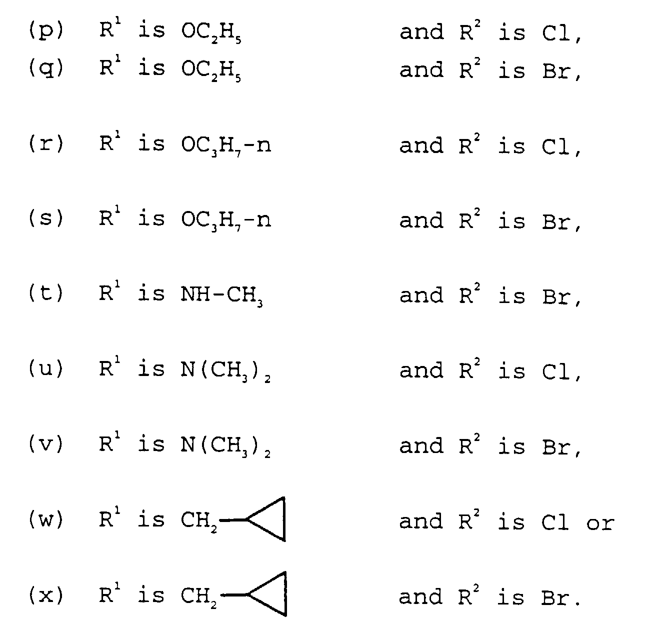

## Revendications

1. Triazolinones halogénées répondant à la formule (II) 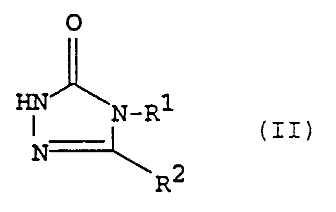 dans laquelle
R¹ représente un groupe alcényle en C₃-C₆, un groupe cycloalkyle en C₃-C₆ portant éventuellement un ou plusieurs substituants identiques ou différents alkyle en C₁-C₄; un groupe alcoxy en C₁-C₆; un groupe alkyl(en C₁-C₄)amino; ou un groupe di-(alkyl en C₁-C₄)amino; et
R² représente un atome de fluor, un atome de chlore, un atome de brome ou un atome d'iode.

2. Triazolinones halogénées de formule (II) selon la revendication 1, caractérisées en ce que, dans cette dernière,
R¹ représente un groupe allyle; un groupe cycloalkyle en C₃-C_{6;} un groupe alcoxy en C₁-C₄; un groupe alkyl(en C₁-C₃)amino; ou un groupe di-(alkyl en C₁-C₃)amino; et
R² représente un atome de chlore ou un atome de brome.

3. Triazolinones halogénées répondant à la formule (II) 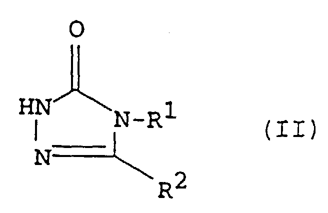 dans laquelle
